(19) Europäisches Patentamt
European Patent Office
Office européen des brevets

(11) **EP 4 616 870 A1**

(12) **EUROPEAN PATENT APPLICATION**
published in accordance with Art. 153(4) EPC

(43) Date of publication:
**17.09.2025 Bulletin 2025/38**

(21) Application number: 23889008.1

(22) Date of filing: **30.10.2023**

(51) International Patent Classification (IPC):
*A61L 2/22* (2006.01)      *A61L 2/20* (2006.01)
*A61L 9/14* (2006.01)      *A61L 9/22* (2006.01)
*B05B 7/04* (2006.01)      *B05B 7/24* (2006.01)
*B05B 5/025* (2006.01)     *B05B 5/03* (2006.01)
*B05B 5/16* (2006.01)

(52) Cooperative Patent Classification (CPC):
A61L 2/20; A61L 2/22; A61L 9/14; A61L 9/22;
B05B 5/025; B05B 5/03; B05B 5/16; B05B 7/04;
B05B 7/24

(86) International application number:
**PCT/KR2023/017013**

(87) International publication number:
**WO 2024/101746 (16.05.2024 Gazette 2024/20)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 07.11.2022  KR 20220146727
           15.06.2023  KR 20230076914

(71) Applicant: **Industry Academic Cooperation
Foundation
of Yeungnam University
Gyeongsan-si, Gyeongsangbuk-do 38541 (KR)**

(72) Inventor: **BYEON, Jeong Hoon
Gyeongsan-si Gyeongsangbuk-do 38541 (KR)**

(74) Representative: **Eisenführ Speiser
Patentanwälte Rechtsanwälte PartGmbB
Postfach 10 60 78
28060 Bremen (DE)**

(54) **DROPLET-PRODUCING DEVICE, AND DISINFECTION SYSTEM COMPRISING SAME**

(57)      The present application provides a droplet-producing device and a disinfection system comprising the droplet-producing device, the device being environmentally friendly and capable of producing charged droplets that can have an antibacterial effect on a space while being harmless to the human body.

[FIG. 1]

EP 4 616 870 A1

**Description**

[Technical Field]

The present application relates to a droplet-producing device and a disinfection system including the same.

[Background Art]

Some of the bacteria and viruses that commonly inhabit the environment in which humans live are pathogens that cause various diseases. In particular, some pathogens contained in food cause food poisoning, and some pathogens are transmitted through the air and cause many casualties in a short period of time.

For example, Norovirus is a food poisoning virus that is transmitted through food, and Norovirus infection is accompanied by vomiting, diarrhea, and dehydration, Ebola virus is transmitted in direct contact with human body fluids, secretions, blood, and the like, and when infected with the Ebola virus, sudden fever, headache, and muscle pain occur, followed by general weakness, skin rash, and systemic bleeding, and Yersinia pestis is transmitted to humans by fleas that live on their host animals, rats, and when infected with the Yersinia pestis, symptoms progress to sudden fever, muscle pain, headache or vomiting, diarrhea or cough, chest pain, and the like.

Therefore, when a person infected with a disease-causing bacteria or virus occurs, a region around the infected person has to be quickly controlled to prevent the bacteria and virus from spreading to the surrounding region, while also quickly sterilizing any remaining bacteria and viruses in the infected region.

Conventional disinfection methods for sterilizing bacteria and viruses include spray disinfection and smoke disinfection methods. The spray disinfection method is a method of mixing chemicals with water and spraying them, and a spraying area is small, but the spray disinfection minimizes environmental pollution and has a residual effect, so it has a high pesticide effect. The smoke disinfection method is used for a wide spraying area and disinfectant particles can reach deep into regions with obstructed airflow, such as wooded regions, but there is no residual effect.

Disinfectors that use these conventional disinfection methods have problems of high cost and environmental pollution because the disinfectors spray chemicals in an aerosol state or make smoke.

[Detailed Description of Invention]

[Technical Problem]

The present invention is directed to providing a droplet-producing device capable of producing charged droplets that are environmentally friendly, harmless to the human body, and capable of spatial antibacterial properties, a method of manufacturing the droplet-producing device, and a disinfection system including the droplet-producing device.

[Technical Solution]

In order to solve the problem, according to one embodiment of the present application, there is provided a droplet-producing device including a housing having an internal space, a droplet discharge unit configured to dropletize a droplet raw material and discharge droplets into the internal space, an electrode tip configured to generate positive or negative ions, an air supply unit configured to supply air toward the electrode tip, and an ion wind injection unit configured to inject positive or negative ion wind formed by mixing the supplied air and the positive or negative ions toward the droplets discharged into the internal space.

[Advantageous Effects]

The droplet-producing device of the present application can produce charged droplets that are environmentally friendly, harmless to the human body, and capable of spatial antibacterial properties.

[Description of Drawings]

FIGS. 1 to 4 are exemplary diagrams showing a droplet-producing device according to one embodiment of the present application.
FIGS. 5 and 6 are exemplary diagrams showing a droplet discharge unit according to one embodiment of the present application.

FIG. 7 is an exemplary diagram showing an electrode tip according to one embodiment of the present application.

FIG. 8 is a scanning electron microscope (SEM) picture showing an untreated electrode tip.

FIG. 9 is an SEM picture showing an electrode tip on which a second protrusion is formed using an etching method.

FIG. 10 is an SEM picture showing an electrode tip on which a second protrusion and a coating portion are formed using an etching method and a deposition method.

[Modes of the Invention]

Hereinafter, a pathogen particle capture device of the present application will be described with reference to the attached drawings, the attached drawings are exemplary, and a droplet-producing device of the present application is not limited to the attached drawings.

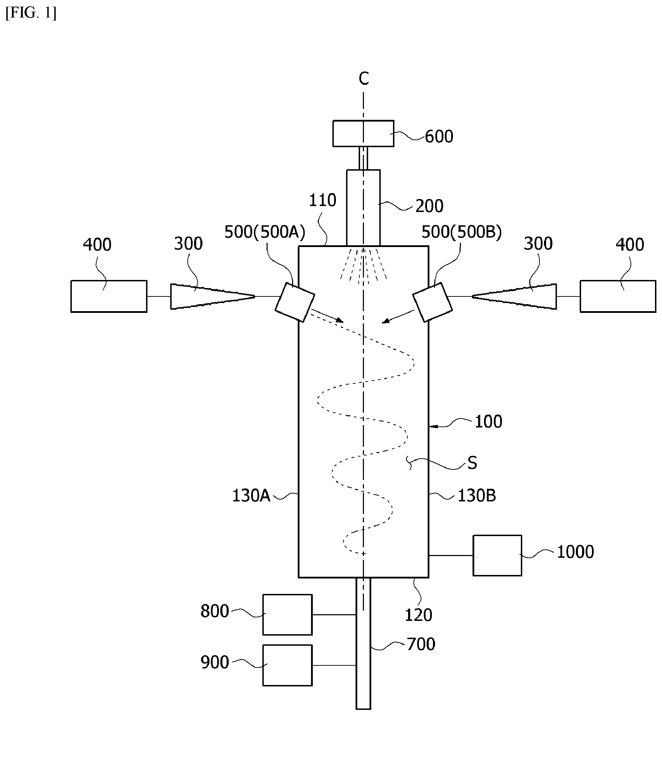

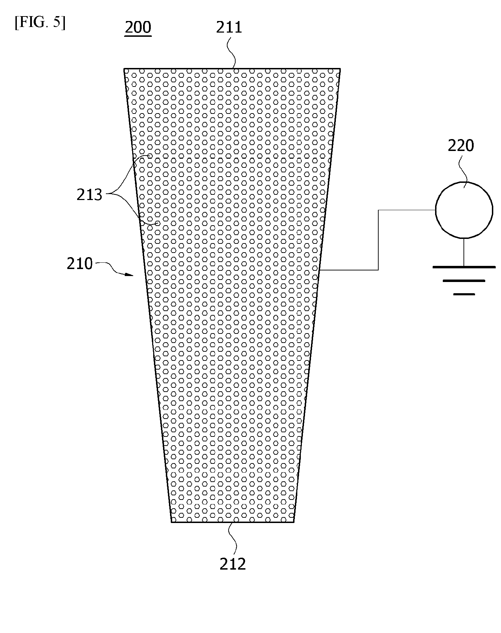

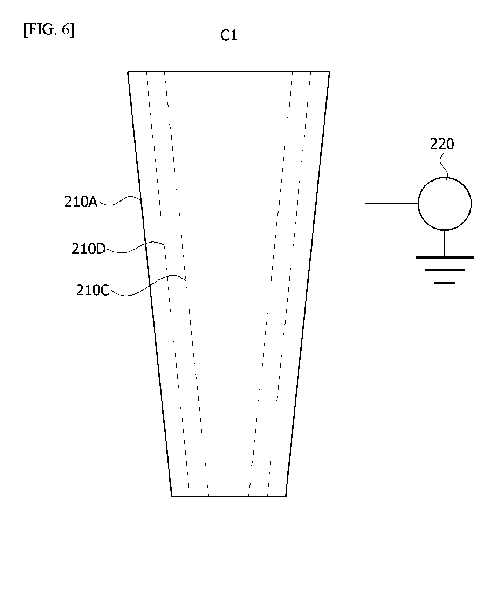

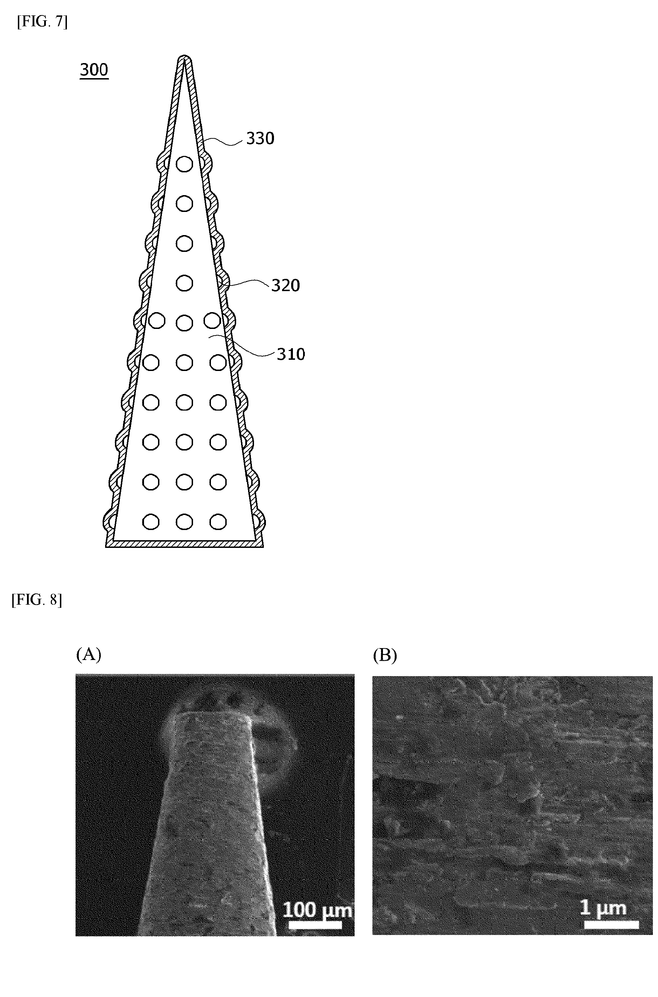

FIGS. 1 to 4 are exemplary diagrams showing a droplet-producing device according to one embodiment of the present application, FIGS. 5 and 6 are exemplary diagrams showing a droplet discharge unit according to one embodiment of the present application, and FIG. 7 is an exemplary diagram showing an electrode tip according to one embodiment of the present application.

Referring to FIG. 1, the droplet-producing device of the present application includes a housing 100, a droplet discharge unit 200, an electrode tip 300, an air supply unit 400, and an ion wind injection unit 500.

The housing 100 has an internal space S. The housing 100 provides the internal space S in which, for example, a droplet comes into contact with an ion wind to form a charged droplet.

The housing 100 has a cylindrical shape and may have openings at an upper portion, a lower portion, and a side portion thereof. The droplet discharge unit 200 may be connected to the opening formed in the upper portion, a discharge tube 700 may be connected to the opening formed in the lower portion, and the ion wind injection unit 500 may be connected to the opening formed in the side portion. However, in the case of the droplet discharge unit 200, according to a discharge method, the droplet discharge unit 200 may be connected to the lower portion or the side portion other than the upper portion.

The droplet discharge unit 200 dropletizes a droplet raw material supplied to the internal space S and discharges droplets. The droplet discharge unit 200 may dropletize the supplied droplet raw material into droplets having a size ranging from tens of nanometers to hundreds of micrometers and then may discharge (or spray) the droplets. The droplet discharge unit 200 may dropletize the droplet raw material using ultrasonic, vibration, or mechanical or electrostatic spraying.

The present application may include a droplet raw material supply unit 600 that stores a droplet raw material and supplies the stored droplet raw material to the droplet discharge unit 200. The droplet raw material may include an antibacterial substance or water that is harmless to the human body. The droplet raw material supply unit 600 may include a supply unit for quantitatively supplying the droplet raw material, and for example, the droplet raw material supply unit 600 may quantitatively supply the droplet raw material through a feed pump, a peristaltic pump, or a syringe pump.

In one example, the droplet discharge unit 200 may be provided at an upper portion 110 of the housing 100. Alternatively, the droplet discharge unit 200 may be provided at a location directed to an upper portion of the internal space S.

As another example, the droplet discharge unit 200 may be provided at a lower portion 120 of the housing 100. Alternatively, the droplet discharge unit 200 may be provided at a location directed to a lower portion of the internal space S.

As still another example, the droplet discharge unit 200 may be provided on a side portion 130 of the housing 100. Alternatively, the droplet discharge unit 200 may be provided at a location directed to a side portion of the internal space S.

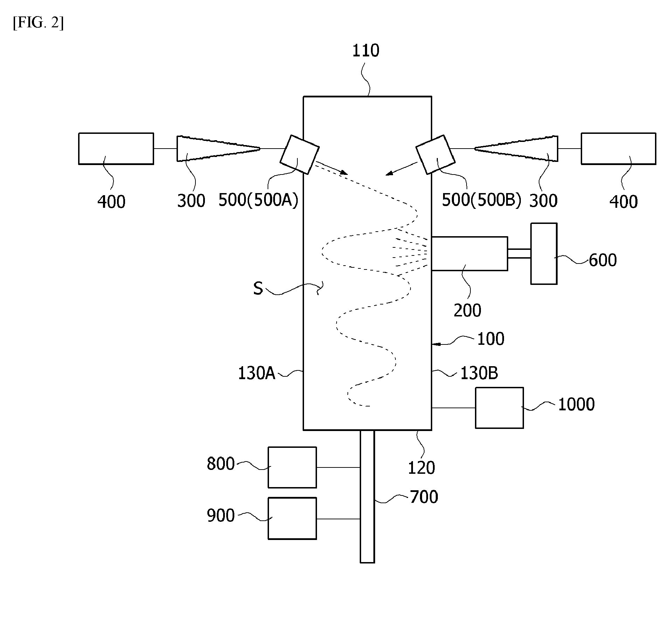

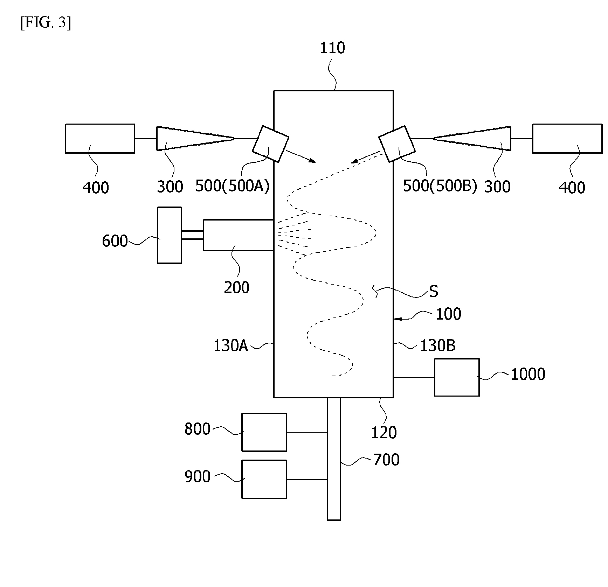

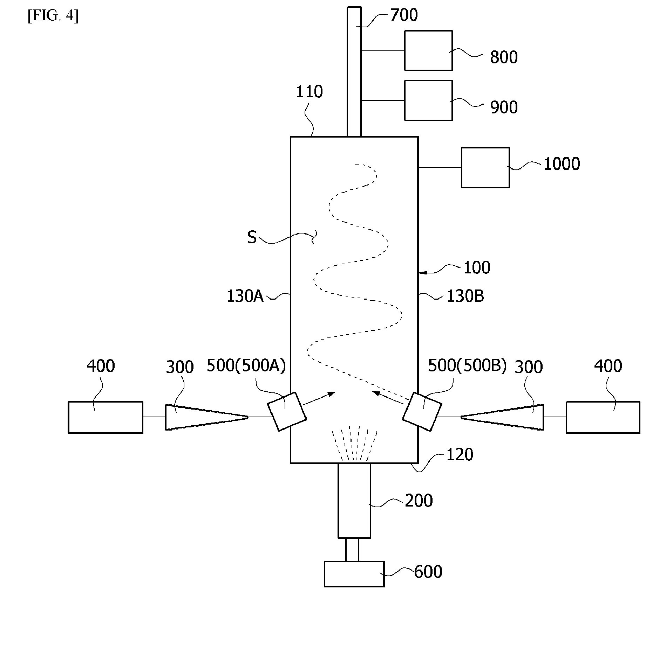

For example, FIG. 1 is an example drawing in which the droplet discharge unit 200 is provided at the upper portion 110 of the housing 100, FIGS. 2 and 3 are exemplary drawings in which the droplet discharge unit 200 is provided at the side portion 130 of the housing 100, and FIG. 4 is an exemplary drawing in which the droplet discharge unit 200 is provided at the lower portion 120 of the housing 100.

According to a location of the droplet discharge unit 200, droplets may be discharged in a downward droplet discharge, upward droplet discharge, or left-rightward droplet discharge manner.

As shown in FIG. 1, when the droplet discharge unit 200 is provided at the upper portion 110 of the housing 100, the droplet discharge unit 200 may discharge droplets in a downward droplet discharge manner. In the downward droplet discharge, the droplet discharged from the droplet discharge unit 200 may move from the upper portion 110 to the lower portion 120 of the housing 100.

As shown in FIGS. 2 and 3, when the droplet discharge unit 200 is provided on side portions 130A and 130B of the housing 100, the droplet discharge unit 200 may discharge droplets in the left-rightward droplet discharge manner. When the droplet discharge unit 200 is provided on the right portion 130B as shown in FIG. 2, droplets may be discharged in a leftward droplet discharge manner, and when the droplet discharge unit 200 is provided on the left portion 130A as shown in FIG. 3, droplets may be discharged in a rightward droplet discharge manner. In the left-rightward droplet discharge, the droplets discharged from the droplet discharge unit 200 may move from the left to the right of the housing 100 or from the right to the left thereof.

As shown in FIG. 4, when the droplet discharge unit 200 is provided at the lower portion 120 of the housing 100, the droplet discharge unit 200 may discharge droplets in the upward droplet discharge manner. In the upward droplet discharge, the droplets discharged from the droplet discharge unit 200 may move from the lower portion 120 to the upper portion 110 of the housing 100.

According to the location of the droplet discharge unit 200, locations of the ion wind injection unit 500 and the discharge tube 700 may be determined.

Specifically, the location of the ion wind injection unit 500 may be provided at a location at which ion wind may be injected in a tangential direction to a discharge direction of the droplet discharge unit 200. For example, when the droplet discharge unit 200 is located at the upper portion 110 or the lower portion 120 of the housing 100, the ion wind injection unit 500 may be located at the side portions 130A and 130B, and when the droplet discharge unit 200 is located at the side portions 130A and 130B of the housing 100, the ion wind injection unit 500 may be located at the upper portion 110 or the lower portion 120.

Additionally, when the droplet discharge unit 200 is located at the upper portion 110, a discharge tube 700 may be located at the lower portion 120, and when the droplet discharge unit 200 is located at the lower portion 120, the discharge tube 700 may be located at the upper portion 110. Additionally, when the droplet discharge unit 200 is located at the side portions 130A and 130B, the discharge tube 700 may be located at the upper portion 110, the lower portion 120, or the side portions 130A and 130B facing the droplet discharge unit 200. For example, when the droplet discharge unit 200 is located at the right portion 130B, the discharge tube 700 may be located at the left portion 130A, and when the droplet discharge unit 200 is located at the left portion 130A, the discharge tube may be located at the right portion 130B.

The present application may include a guide vane that controls a discharge angle of the droplet discharge unit 100. By finely controlling the discharge angle of the droplet discharge unit 100 through the guide vane, the droplets may be discharged in a desired direction.

In addition, a plurality of droplet discharge units 100 may be provided. The droplet-producing device according to the present application may include a droplet discharge control unit that controls the droplet discharge of the plurality of droplet discharge units 100. The droplet discharge control unit may control an amount of droplets discharged into the housing 100 by controlling the discharge of droplets from each of the plurality of droplet discharge units 100. In addition, the droplet discharge control unit may control a flow rate of the droplet raw material. The droplet discharge control unit may control an amount of discharge of the droplets by controlling the flow rate of the droplet raw material.

Referring to FIG. 5, the droplet discharge unit 200 may include a nozzle body 210 having an inlet 211 through which the droplet raw material is introduced and a nozzle tip 212 that discharges the introduced droplet raw material as droplets; and a power supply unit 220 that applies a voltage to electrostatically charge droplets discharged through the nozzle tip 212. The power supply unit 220 may apply a voltage to the nozzle body 210 or apply a voltage to a separate electrode (not shown) connected to the nozzle body 210 so that the atomized droplets are electrostatically charged and discharged.

The nozzle body 210 may have a droplet space in which the introduced droplet raw material is dropletized.

As shown in FIG. 6, the nozzle body 210 may include one nozzle body unit or a plurality of nozzle body units 210A, 210B and 210C overlappingly fitted to each other with respect to a common axis C1. Since the nozzle body 210 includes the plurality of nozzle body units overlappingly fitted to each other with respect to the common axis C1, it is easy to control a composition, size, and shape of the droplets, and also it is easy to control a adjustment range of the discharge flow rate and speed. Although FIG. 6 exemplarily shows three nozzle body units, the invention is not limited thereto, and the nozzle body 310 may include 2 to 10 nozzle body units.

Preferably, the nozzle body 210 is manufactured from a conductive material so that a current flows therethrough due to the applied voltage. For example, the nozzle body 102 may include a transition metal such as iron, tungsten, silver, copper, gold, nickel, cobalt, zinc, molybdenum, or an alloy thereof. Alternatively, the nozzle body 102 may include stainless steel.

Additionally, the nozzle body 210 may include first protrusion portions 213 each having a nano-size and formed on a surface thereof. For example, when the nozzle body 310 includes the plurality of nozzle body units 210A, 210B and 210C, the first protrusion portions 213 may be formed on a surface of each of the plurality of nozzle body units. The first protrusion portions 213 may be formed by an etching or etching-chemical vapor deposition (CVD) treatment. Since the nozzle body 210 includes the first protrusion portions 213 on the surface, it is possible to lower the applied voltage for electrostatic spraying or increase an amount of charge of the droplet with respect to the same voltage and also have an effect of suppressing generation of by-products such as ozone.

The electrode tip 300 generates positive or negative ions. The positive or negative ions generated at the electrode tip may be mixed with air supplied from the air supply unit 400 described below and discharged to the side of the ion wind injection unit 500.

More specifically, the electrode tip 300 may include an electrode body 310, a second protrusion portions 320, and a coating portion 330.

The electrode body 310 is a part that becomes a body of an electrode. In one example, the electrode body 310 may have a pin shape. Since the electrode body 310 has the pin shape, an active area may be expanded when ions are generated, and at the same time, an ionization discharge onset voltage for ion generation may be lowered, thereby suppressing generation of ozone which is a by-product that may occur during the ion generation.

The electrode body 310 may be made of a material for electrodes commonly used in the art. Specifically, the electrode body 310 may include a transition metal such as iron, tungsten, silver, copper, gold, nickel, cobalt, zinc, molybdenum, or an alloy thereof.

The second protrusion portion 320 may be a protrusion portion having a nano-size and formed on a surface of the electrode body 410. In this specification, "nano" may mean a size in nanometers (nm), for example, is a size of 0.1 nm to 1,000 nm, but is not limited thereto. Additionally, in this specification, "nano-pin" means that a protrusion having an average diameter in nanometers (nm) is formed on a surface of a body having a pin shape. In addition, in this specification, a "pin" may mean a structure having a rod shape of which a length is greater than a cross-sectional area, and a diameter becomes smaller in a direction toward the side of an end portion, and thus having a pointed shape.

The second protrusion portion 320 is a portion that protrudes from the surface of the electrode body 410, is formed on the surface of the electrode body 310, and may have a nano size. Since the electrode tip 300 has the nano-sized second protrusion portions 320 on the surface of the electrode body 310, it is possible to induce a shape in which the ionization discharge onset voltage required for ion generation is lowered, when ions are generated, ions distributed on the surfaces of the electrode body 310 and the second protrusion portions 320 are dispersed, and thus, due to a reduced impulse caused by a low electron movement speed, ozone production is suppressed by causing outer electrons of oxygen atoms to be mainly removed rather than oxygen dissociation, and an amount of ion generation increases. As a consequence, the electrode tip 310 may maintain a residual ozone concentration below an indoor standard.

The plurality of second protrusion portions 320 may be formed on the surface of the electrode body 310, but the number of the plurality of second protrusion portions 320 is not particularly limited. **In** this specification, the term "plurality" means two or more, and the upper limit is not particularly limited.

In one example, the second protrusion portion 320 may have a radius of curvature of 1 nm to 10 $\mu$m. Specifically, the radius of curvature of the second protrusion portion 320 may range from 5 nm to 8 $\mu$m, 10 nm to 6 $\mu$m, 50 nm to 4 $\mu$m, or 100 nm to 2 $\mu$m. Since the second protrusion portion 320 has a radius of curvature within the above-described range, it is possible to lower an ionization discharge onset voltage for generating negative ions and thus lower an electric field intensity, thereby suppressing ozone generation.

For example, in the electrode body 310, the ionization discharge onset voltage for ion generation may range from 0.02 kV to 20 kV, and specifically, may range from 0.05 kV to 18 kV, 0.1 kV to 15 kV, 0.5 kV to 13 kV, or 1 kV to 10 kV. The electrode may suppress ozone generation by satisfying the ionization discharge onset voltage for ion generation within the above-described range and lowering the electric field intensity.

In this case, the ionization discharge onset voltage $V_s$ for ion generation may be calculated by the following Equation 1.

[Equation 1]

$$V_S = \frac{r}{2} \times E \times \ln\left[\frac{r + 2d}{r}\right]$$

In Equation 1, r is a radius of curvature of a protrusion portion, E is an electric field intensity when ionization begins to appear on the surfaces of the electrode body and the protrusion for ion generation, and d is a distance between the electrode and a ground plate. In this case, the electric field intensity E may be calculated by substituting the ionization discharge onset voltage $V_s$ obtained through an actual experiment, the radius of curvature r of the already specified protrusion portion, and the distance d between the electrode and the ground plate.

The distance d between the electrode body 310 and the ground plate may range from 4 mm to 16 mm in the air, and specifically, the lower limit may be 6 mm or more, 8 mm or more, or 10 mm or more, and the upper limit may be 14 mm or less or 12 mm or less. When the distance between the electrode and the ground plate satisfies the above-described range, the applied voltage for ion generation is lowered, thereby lowering the electric field intensity and suppressing ozone generation. However, when the distance between the electrode and the ground exceeds the above-described range, the applied voltage for negative ion generation may increase, which may result in a disadvantage in which the electric field intensity is increased and ozone generation is increased.

In one example, the second protrusion portions 320 are integrated with the electrode body 310 by etching, impregnation, and spraying processes described below and may be made of the same material as that of the electrode body 310. For example, the protrusion portion 320 may include a transition metal such as iron, tungsten, silver, copper, gold, nickel, cobalt, zinc, molybdenum, or an alloy thereof.

The coating portion 330 is a portion formed by coating the surfaces of the electrode body 310 and the second protrusion portions 320 through a deposition process described below, and is a portion formed by coating conductive carbon on the above-described surfaces. The electrode tip 300 including the coating portion 330 coated with conductive carbon on the above-described surfaces can prevent corrosion of the electrode and exhibit excellent antibacterial performance.

As an example, the coating portion 330 may be formed in the form of a film or fiber on the surfaces of the electrode body 310 and the second protrusion portions 320. The coating portion 330 having the above-described form can prevent corrosion of the electrode and exhibit excellent antibacterial performance.

The conductive carbon is carbon having a conductive property and may be included in the electrode in an amount of 10 to 40 parts by weight with respect to 100 parts by weight of the transition metal. Specifically, the conductive carbon may be included in the electrode tip 300 in an amount of 13 to 38 parts by weight, 15 to 35 parts by weight, 18 to 33 parts by weight, 20 to 30 parts by weight, 23 to 28 parts by weight, or 25 to 28 parts by weight with respect to 100 parts by weight of the transition metal. When the conductive carbon is included in the electrode tip 300 in the above-described content, an ion generation concentration is excellent, a residual ozone concentration is maintained below an indoor standard value, and excellent antibacterial performance can be exhibited.

In one example, the electrode tip 300 may have an ion generation concentration of $8 \times 10^5$ ions/cm$^3$ or more. The ion generation concentration of the electrode may be specifically $9 \times 10^5$ ions/cm$^3$ or more, $10 \times 10^5$ ions/cm$^3$ or more, $11 \times 10^5$ ions/cm$^3$ or more, or $12 \times 10^5$ ions/cm$^3$ or more. Additionally, the upper limit of the ion generation concentration in the electrode measured under the above-described conditions may be $1 \times 10^8$ ions/cm$^3$ or less, $5Y10^7$ ions/cm$^3$ or less, $1 \times 10^7$ ions/cm$^3$ or less, $5 \times 10^6$ ions/cm$^3$ or less, $4 \times 10^6$ ions/cm$^3$ or less, $35 \times 10^5$ ions/cm$^3$ or less, or $33 \times 10^5$ ions/cm$^3$ or less.

The electrode tip 300 may have an excellent ion generation concentration and maintain a residual ozone concentration below the indoor standard value by satisfying the above-described range of the ion generation concentration. In this case, an ion measurement unit is installed at a certain distance from a part in which the ions are generated, and thus the generated ions may be measured after sufficiently diffusing in the air, thereby increasing measurement reliability.

The electrode tip 300 may generate negative or positive ions by applying a negative DC voltage or a positive DC voltage to the electrode body 410. For example, a positive DC voltage or a negative DC voltage of 7 kV may be applied to generate positive or negative ions.

In detail, the second protrusion portions 320 and the coating portion 330 of the electrode tip 300 may be formed by the following methods.

1) Liquid etching method: A liquid etching method may include, for example, a method of forming the second protrusion portions 320 by immersing the electrode body 310 in an etching reagent (also called a corrosive solution or etchant). The liquid etching method may control physical factors such as etching reagent stirring speed, temperature, and ultrasonic application to control etching intensity, speed, and shape, and may also perform liquid etching electrochemically by generating an electric field for etching.

2) Dry etching method: A dry etching method may include, for example, a method of forming the second protrusion portions 320 on the surface by applying sputtering in which surface components are detached by colliding the electrode body 310 with high-energy electrons or ions, a method of forming the second protrusion portions 320 on the surface by ablating surface components as high-energy photoirradiation is applied to the surface of the electrode body 310, and a method of forming the second protrusion portions 320 on the surface of the electrode body 310 by mechanical polishing

3) Spraying method: A spraying method may include, for example, a method of spraying a solution in which a precursor material for the second protrusion portions 320 is dispersed into droplets to form the second protrusion portions 320 on the surface of the electrode body 310.

4) Deposition method: A deposition method may include, for example, a CVD method, and the deposition method may be used as a method of forming the coating portion 330 and provide an additional function to the electrode body 310 on which the second protrusion portions 320 are formed. For example, a carbon-based conductive material may be deposited on the coating portion 330 to increase an amount of ion emission in the electrode tip 300 or to provide moisture formation preventing or corrosion-proofing functions to the tip.

Etching, spraying, and deposition materials (or solutions) use the material of the second protrusion portion 320 or the coating portion 330 as a base material and may include additives such as a hydrophobic coating agent to prevent moisture formation on the surface of the electrode tip 300 in addition to the base material, and a fixing component to increase fixing strength with the surface of the electrode tip 300.

In addition, after the second protrusion portions 320 are formed, heat treatment (sintering or thermal curing) may be performed to increase an electrode tip fixing force between the second protrusion portions 320 and the electrode body 310.

In one embodiment, an experiment was conducted to check whether the second protrusion portions 320 and the coating portion 330 were formed on the surface of the electrode body 310 using the above-described methods.

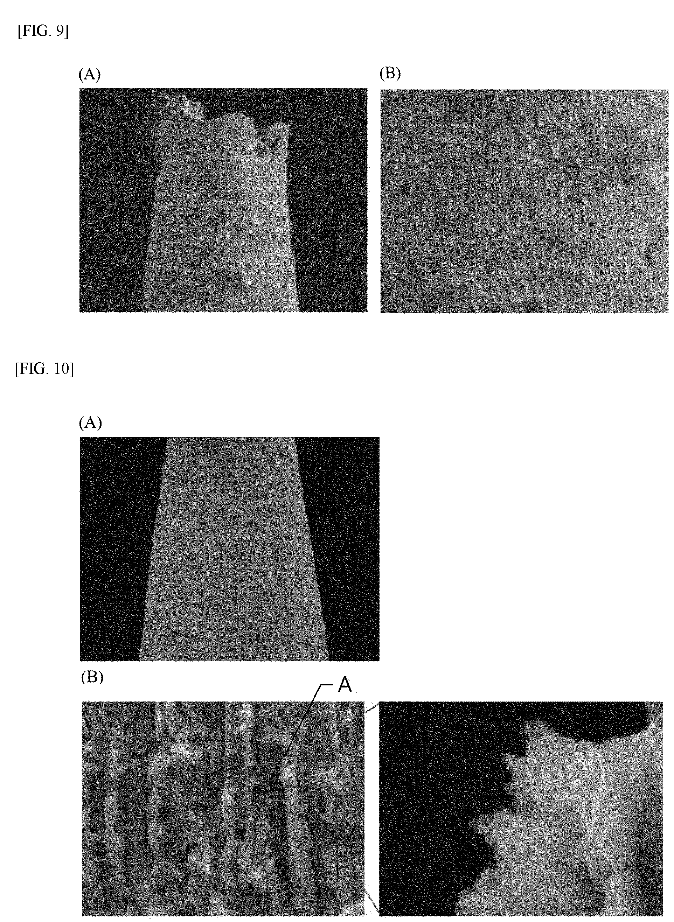

FIG. 8 is a scanning electron microscope (SEM) picture showing an untreated electrode tip, FIG. 9 is an SEM picture showing an electrode tip on which a protrusion is formed using the etching method, and FIG. 10 is an SEM picture showing an electrode tip on which a protrusion and a coating portion are formed using the etching method and the deposition method.

FIGS. 8A, 9A, and 10A are 500-magnification SEM pictures, FIGS. 8B and 9B are 10,000-magnification SEM pictures, and a left picture of FIG. 10B is a 10,000-magnification SEM picture, and a right picture is a 50,000-magnification SEM picture of a "region A" in the left picture.

Specifically, FIG. 9 is a picture of an electrode tip of the electrode body 310 on which the second protrusion portions 320 are formed by immersing in a tungsten etching solution for 5 minutes under 40 kHz ultrasonic conditions. FIG. 10 is a picture showing an electrode tip electrode body 410 formed by immersing the electrode body in a tungsten etching solution for 5 minutes under 40 kHz ultrasonic conditions to form the second protrusion portions 320, and then performing the CVD in an acetylene and nitrogen environment (volume ratio 1:5) at 650° C and 3 Torr.

Referring to FIGS. 8 to 10, it was confirmed that it was possible to manufacture the electrode tip 300 in which the second protrusion portions 320 and the coating portion 330 are formed on the surface of the electrode body 310 using the various above-described methods.

Meanwhile, the air supply unit 400 supplies air toward the electrode tip 300. The electrode tip 300 may be provided on the air supply line of the air supply unit 400. The air may be mixed with positive or negative ions generated at the electrode tip 300 to form ion wind. The ion wind may mean that ions are present in a suspended state in the air. The air supply unit 400 may include a speed control unit that controls a supply speed of the air. The speed control unit may control an injection speed of the ion wind by controlling the supply speed of the air.

The ion wind injection unit 500 injects positive or negative ion wind formed by mixing supplied air and positive or negative ions toward droplets discharged into the housing 100. The ion wind injection unit 500 may include an inlet unit through which the positive or negative ion wind is introduced and an outlet unit connected to a side opening of the housing 100 to discharge the introduced positive or negative ion wind toward droplets discharged within the housing 100.

In one specific example, the ion wind injection unit 500 may include a first ion wind injection unit 500A that injects positive ion wind toward the droplets discharged into the internal space S, and a second ion wind injection unit 500B that injects negative ion wind toward the droplets discharged into the internal space S.

The droplet-producing device according to the present invention may control the polarity of the charged droplets through the first ion wind injection unit 500A and the second ion wind injection unit 500B that inject ion winds having different polarities.

The housing 100 includes a first region into which positive ion wind is injected by the first ion wind injection unit 500A and a second region into which negative ion wind is injected by the second ion wind injection unit 500B, and the first and second regions may be separated from each other. In this case, the droplet discharge unit 200 may include a first droplet discharge unit that discharges droplets to the first region and a second droplet discharge unit that discharges droplets to the second region. The droplet-producing device according to the present invention may manufacture droplets each charged with different polarities in separated spaces.

In one specific example, the ion wind injection unit 500 may inject positive or negative ion wind so that the positive or negative ion wind circles the internal space S. In other words, the ion wind injection unit 500 may inject positive or negative ion wind so that the positive or negative ion wind circles along an inner surface of the housing 100 facing the internal space S. The positive or negative ion wind injected to circle may move toward the discharge tube 700 while circling in a circumferential direction of the internal space S.

For example, the droplet discharge unit 200 may be provided to discharge droplets toward the discharge tube 700 in a direction of the central axis C of the housing 100, and the ion wind injection unit 500 may be provided so that positive or negative ion wind is injected along an imaginary injection line having a predetermined slope with respect to the direction of the central axis C of the housing 100. The ion wind injection unit 500 may inject positive or negative ion wind so that the positive or negative ion wind is introduced in a tangential direction to the inner surface of the housing 100. The positive or negative ion wind may move downward in the tangential direction to the inner surface of the housing 100 toward the discharge tube 700. The ion wind that is injected to circle may have an advantage of high charging efficiency because frequency of contact with the discharged droplets is maximized.

In one example, the discharged droplets may be charged into positive or negative ion droplets by coming into contact with the injected positive ion or negative ion wind. For example, when only the first ion wind injection unit 500A is operated, the discharged droplets come into contact with the positive ion wind and are charged with a positive (or positive, +) polarity, and when only the second ion wind injection unit 500B is operated, the discharged droplets come into contact with the negative ion wind and are charged with a negative (or negative, -) polarity. In addition, when the first and second ion wind injection units 500A and 500B are operated simultaneously, some of the discharged droplets may come into contact with the positive ion wind and be charged with + polarity, and some may come into contact with the negative ion wind and be charged with - polarity. In particular, droplets that are charged with both polarities (+ and -) may have better antibacterial efficacy than droplets that are charged with a single polarity (+ or -).

The droplet-producing device of the present application may include the discharge tube 700 for discharging the charged droplets. The charged droplets may be supplied through the discharge tube 700 to a location at which space disinfection is required.

The discharge tube 700 may discharge positive ion droplets and negative ion droplets separately or together. In addition, the discharge tube 700 may discharge positive ion wind and negative ion wind separately or together. The discharge tube 700 may discharge the droplets and the ion wind singly, together, or alternately.

In addition, a discharge amount control unit for controlling an amount of discharge in the discharge tube 700 may be additionally included. The discharge amount control unit may control the amounts of discharge of the droplets and the ion wind.

The discharge tube 700 includes a first discharge tube through which positive ion droplets move and a second discharge tube through which negative ion droplets move, and the first and second discharge tubes may be separated. The first discharge tube 700 may be connected to the first region of the housing 100, and the second discharge tube may be connected to the second region of the housing 100. The first region of the housing 100 may be a region into which positive ion wind is injected by the first ion wind injection unit 500A, and the second region of the housing 100 may be a region into which negative ion wind is injected by the second ion wind injection unit 500B.

In one example, the discharge tube 700 may be hydrophobically surface-treated. An inner surface of the discharge tube 700 that may come into contact with the droplets may be hydrophobically surface-treated. The hydrophobic surface treatment may be intended to prevent moisture formation, condensation, and corrosion caused by charged droplets within the discharge tube 700.

For example, the discharge tube 700 may include a moisture absorption column or a heating means. The moisture absorption column or heating means may serve to prevent moisture formation due to charged droplets within the discharge tube when the droplet-producing device is used for a long time. In addition, the moisture absorption column or heating means may promote evaporation of charged droplets in the discharge tube 700 to enable supply centered on radicals or ions.

In one specific example, the droplet-producing device according to the present invention may include an electric field applying unit 800 for applying an electric field to the discharge tube 700, and a magnetic field applying unit 900 for applying a magnetic field to the discharge tube 700. The electric field applying unit may apply an electric field to the discharge tube 700 to cause the charged droplets to move through the discharge tube 700 in the form of a water spray or jet spray. In addition, the magnetic field applying unit may accelerate a flow speed of the droplets by applying a magnetic field to the discharge tube 700.

In addition, the droplet-producing device according to the present invention may include a collection unit 1000 that collects condensate water formed by droplets discharged into the internal space S and attached to the inner surface of the housing 100 facing the internal space S. The condensate water may be a factor that deteriorates the performance of the droplet-producing device. The collection unit 1000 can prevent the performance of the droplet-producing device from deteriorating by collecting the condensate water. The collection unit 1000 may include a drain unit that drains the collected condensate water.

In one example, the air supply unit 400 may include a filter. The air supply unit 400 may supply clean air through the filter, and the filter may be a high-efficiency particulate air (HEPA) filter but is not limited thereto.

The droplet-producing device according to the present invention may be, for example, a device for producing droplets provided with a disinfection function. The droplets provided with the disinfection function may be supplied to a location at which disinfection is required and may serve to remove sources of contamination such as bio-aerosols.

In one example, the droplet raw material may include water or an antibacterial substance.

The antibacterial substance may include, for example, a generally recognized as safe (GRAS) substance designated by the United States Food and Drug Administration (USFDA).

The GRAS substance may include, for example, one or more selected from a group consisting of metal chlorides, metal oxides, metal nitrides, and organic antibacterial substances.

Examples of the metal chlorides include sodium chloride (NaCl), potassium chloride (KCl), magnesium chloride ($MgCl_2$), and the like.

Examples of the metal oxides include magnesium oxide (MgO), zinc oxide (ZnO), copper oxide (CuO), manganese oxide (MnO), iron oxide ($Fe_3O_4$), and the like.

Examples of the metal nitride include silver nitride ($AgNO_3$), magnesium nitride ($Mg(NO_3)_2$), or zinc nitride ($Zn(NO_3)_2$).

Additionally, examples of the organic antibacterial substances include acetic acid, benzoic acid, benzonates, dimethyl dicarbonate, lactic acid, lactates, lactoferrin, lysozyme, nisin, parabens, propionic acid, propionates, sorbic acid, sorbates, cetylpyridinium chloride, chlorine dioxide, peroxyacids, and the like.

In addition to the GRAS substances, various known particulate substances or antibiotics exhibiting the antibacterial function can be used as droplet raw materials, and for example, GRAS substances, particulate substances, and antibiotics can be used alone or in combination as droplet raw materials.

Examples of the particulate substances may include Ag, Au, copper, graphene, graphene oxide, carbon nanotube, carbon nanofiber, carbon nanodot, graphitic carbon nitride, boron nitride, Si, silicon oxide, Te, Se, nanoclay, and the like.

Examples of the antibacterial substances include sulfonamides, penicillin, erythromycin, natamycin, vancomycin, methicillin, ampicillin, ciprofloxacin, linezolid, ceftaroline, and the like.

The present application also relates to a disinfection system including the above-described droplet-producing device. The disinfection system can perform space disinfection by discharging or spraying charged droplets produced from the above-described droplet-producing device into a space requiring disinfection.

The disinfection system may include a moving means for moving a droplet-producing device, a sensor that measures a concentration of pollutants within a disinfection site, and a control unit that controls a movement direction of the moving means and an operation of the droplet-producing device based on the sensor. The sensor can measure a concentration of bio-aerosol or microorganism within the disinfection site, and the control unit can move the moving means to a region in which the concentration of pollutants exceeds an acceptable numerical range, and then can operate the droplet-producing device to supply produced droplets to the region. The control unit may enable unmanned operation of the disinfection system.

In addition, the disinfection system according to the present invention may include a data receiving unit that receives data comparing the concentration of pollutants after disinfection with the concentration of pollutants before disinfection and the environmental standard concentration through the sensor that measures the concentration of pollutants. The data receiving unit can receive data via a wired/wireless communication unit and can provide the received data to a user.

An example of a disinfection process of the disinfection system can be carried out in the order of 1) to 6) below.

1) Measure pollutant concentration in a disinfection site
2) Perform comparison operation between measured concentration and environmental standards
3) Transmit whether the disinfection system is operating by the operation
4) When an operation of the disinfection system is decided, the droplet-producing device moves to the disinfection site by a moving means and the droplet-producing device performs disinfection in a mode of droplet-ion wind mixed discharge and droplet-ion wind alternating discharge or droplet discharge followed by ion wind discharge.
5) After disinfection, the concentration of pollutants is measured through a sensor and then data is transmitted.
6) When the pollutant concentration is reduced to an appropriate level, the droplet-producing device is returned to an original position thereof by the moving means, or additional disinfection is performed when further reduction in the pollutant concentration is required, and then the droplet-producing device is returned to the original position thereof by the moving means when the appropriate reduction is made.

[Reference Signs List]

100: Housing
200: Droplet discharge unit
300: Electrode tip
400: Air supply unit
500: Ion wind injection unit

**Claims**

1. A droplet-producing device comprising:

    a housing having an internal space;
    a droplet discharge unit configured to dropletize a droplet raw material and discharge droplets into the internal space;
    an electrode tip configured to generate positive or negative ions;
    an air supply unit configured to supply air toward the electrode tip; and
    an ion wind injection unit configured to inject positive or negative ion wind formed by mixing the supplied air and the positive or negative ions toward the droplets discharged into the internal space.

2. The droplet-producing device of claim 1, wherein the droplet discharge unit is provided on an upper portion of the housing.

3. The droplet-producing device of claim 1, wherein the droplet discharge unit is provided on a lower portion of the housing.

4. The droplet-producing device of claim 1, wherein the droplet discharge unit is provided on a side portion of the housing.

5. The droplet-producing device of claim 1, wherein the droplet discharge unit includes:

   a nozzle body including an inlet through which the droplet raw material is introduced and a nozzle tip that discharges the introduced droplet raw material as droplets; and
   a power supply unit that applies a voltage to electrostatically charge the droplets discharged through the nozzle tip.

6. The droplet-producing device of claim 5, wherein the nozzle body includes one nozzle body unit or a plurality of nozzle body units overlappingly fitted to each other with respect to a common axis.

7. The droplet-producing device of claim 6, wherein the nozzle body includes first protrusion portions each having a nano-size and formed on a surface thereof.

8. The droplet-producing device of claim 1, wherein the electrode tip includes:

   an electrode body;
   second protrusion portions each having a nano-size and formed on a surface of the electrode body; and
   a coating portion coated with conductive carbon on surfaces of the electrode body and the second protrusion portions.

9. The droplet-producing device of claim 1, wherein the ion wind injection unit includes:

   a first ion wind injection unit that injects the positive ion wind toward the droplets discharged into the internal space; and
   a second ion wind injection unit that injects the negative ion wind toward the droplets discharged into the internal space.

10. The droplet-producing device of claim 1, wherein the ion wind injection unit injects the positive or negative ion wind so that the positive or negative ion wind circles the internal space.

11. The droplet-producing device of claim 10, wherein the ion wind injection unit injects the positive or negative ion wind so that the positive or negative ion wind circles along an inner surface of the housing facing the internal space.

12. The droplet-producing device of claim 1, wherein the discharged droplets come into contact with the injected positive or negative ion wind and are charged as positive or negative ion droplets.

13. The droplet-producing device of claim 12, comprising a discharge tube configured to discharge the charged droplets.

14. The droplet-producing device of claim 13, wherein the discharge tube discharges positive ion droplets and negative ion droplets separately or together.

15. The droplet-producing device of claim 13, wherein the discharge tube is subjected to a hydrophobic surface treatment.

16. The droplet-producing device of claim 13, comprising:

   an electric field applying unit configured to apply an electric field to the discharge tube; and
   a magnetic field applying unit configured to apply a magnetic field to the discharge tube.

17. The droplet-producing device of claim 1, comprising a collection unit configured to collect condensate water formed by the droplets discharged into the internal space and attached to an inner surface of the housing facing the internal space.

18. The droplet-producing device of claim 1, wherein the air supply unit includes a filter.

19. The droplet-producing device of claim 1, wherein the droplet raw material includes water or an antibacterial substance.

20. A disinfection system including the droplet-producing device of claim 1.

[FIG. 1]

[FIG. 2]

[FIG. 3]

[FIG. 4]

[FIG. 5]

[FIG. 6]

C1

220

210A

210D

210C

[FIG. 7]

300

[FIG. 8]

(A)                                    (B)

[FIG. 9]

(A)

(B)

[FIG. 10]

(A)

(B)

A

## INTERNATIONAL SEARCH REPORT

| International application No. |
|---|
| **PCT/KR2023/017013** |

| A. | CLASSIFICATION OF SUBJECT MATTER |
|---|---|

**A61L 2/22**(2006.01)i; **A61L 2/20**(2006.01)i; **A61L 9/14**(2006.01)i; **A61L 9/22**(2006.01)i; **B05B 7/04**(2006.01)i; **B05B 7/24**(2006.01)i; **B05B 5/025**(2006.01)i; **B05B 5/03**(2006.01)i; **B05B 5/16**(2006.01)i

According to International Patent Classification (IPC) or to both national classification and IPC

| B. | FIELDS SEARCHED |
|---|---|

Minimum documentation searched (classification system followed by classification symbols)

A61L 2/22(2006.01); A61G 10/00(2006.01); A61L 2/03(2006.01); A61L 2/14(2006.01); A61L 9/14(2006.01); B01D 47/06(2006.01); B05B 5/057(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 액적(droplet), 방역(prevention of epidemics), 항균(antibacterial), 양이온 (cation), 음이온(anion), 전극(electrode), 공기(air), 노즐(nozzle)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2017-0093746 A (IUCF-HYU (INDUSTRY-UNIVERSITY COOPERATION FOUNDATION HANYANG UNIVERSITY)) 16 August 2017 (2017-08-16)<br>See paragraphs [0002], [0033], [0040]-[0042] and [0065]-[0073]; and figures 1 and 4-7. | 1-4,10-11,17-20 |
| Y | | 5-6,12-15 |
| A | | 7-9,16 |
| Y | KR 10-2014-0025345 A (FINISHING BRANDS HOLDINGS INC.) 04 March 2014 (2014-03-04)<br>See paragraphs [0023]-[0026]; and figures 2-3. | 5-6,12-15 |
| Y | WO 2020-012954 A1 (PANASONIC INTELLECTUAL PROPERTY MANAGEMENT CO., LTD.) 16 January 2020 (2020-01-16)<br>See paragraphs [0192] and [0200]; and figures 13-14. | 6 |
| A | KR 10-1924620 B1 (LEE, Seung Jae) 03 December 2018 (2018-12-03)<br>See entire document. | 1-20 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **14 February 2024** | **16 February 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2023/017013** |

### C. DOCUMENTS CONSIDERED TO BE RELEVANT

| Category* | Citation of document, with indication, where appropriate, of the relevant passages | Relevant to claim No. |
|---|---|---|
| A | JP 2000-325727 A (NIKKO SOHONSHA:KK) 28 November 2000 (2000-11-28)<br>See entire document. | 1-20 |

**INTERNATIONAL SEARCH REPORT**
Information on patent family members

International application No.

**PCT/KR2023/017013**

| Patent document cited in search report | | | Publication date (day/month/year) | Patent family member(s) | | | Publication date (day/month/year) |
|---|---|---|---|---|---|---|---|
| KR | 10-2017-0093746 | A | 16 August 2017 | KR | 10-2017-0093745 | A | 16 August 2017 |
| | | | | KR | 10-2019-0024950 | A | 08 March 2019 |
| | | | | KR | 10-2214680 | B1 | 10 February 2021 |
| | | | | US | 10857250 | B2 | 08 December 2020 |
| | | | | WO | 2017-135780 | A1 | 10 August 2017 |
| KR | 10-2014-0025345 | A | 04 March 2014 | AU | 2012-214278 | B2 | 29 January 2015 |
| | | | | BR | 112013020466 | A2 | 08 August 2017 |
| | | | | CA | 2827119 | A1 | 16 August 2012 |
| | | | | CL | 2013002338 | A1 | 12 December 2014 |
| | | | | CN | 103635207 | B | 30 September 2015 |
| | | | | EP | 2673007 | A1 | 18 December 2013 |
| | | | | EP | 2673007 | B1 | 10 February 2016 |
| | | | | EP | 2886133 | A1 | 24 June 2015 |
| | | | | JP | 2014-511228 | A | 15 May 2014 |
| | | | | JP | 5813786 | B2 | 17 November 2015 |
| | | | | MX | 2013009338 | A | 17 February 2014 |
| | | | | RU | 2013140627 | A | 20 March 2015 |
| | | | | US | 2012-0207651 | A1 | 16 August 2012 |
| | | | | WO | 2012-109556 | A1 | 16 August 2012 |
| | | | | ZA | 201306722 | B | 29 October 2014 |
| WO | 2020-012954 | A1 | 16 January 2020 | CN | 112384252 | A | 19 February 2021 |
| | | | | EP | 3821914 | A1 | 19 May 2021 |
| | | | | EP | 3821914 | B1 | 06 December 2023 |
| | | | | JP | 7065357 | B2 | 12 May 2022 |
| | | | | US | 2021-0229120 | A1 | 29 July 2021 |
| | | | | WO | 2020-012954 | A1 | 16 January 2020 |
| KR | 10-1924620 | B1 | 03 December 2018 | None | | | |
| JP | 2000-325727 | A | 28 November 2000 | None | | | |

Form PCT/ISA/210 (patent family annex) (July 2022)